# EUROPEAN PATENT SPECIFICATION

(11) **EP 1 741 447 B1**
(45) Date of publication and mention of the grant of the patent: **18.09.2013**
(21) Application number: 06121896.2
(22) Date of filing: 19.01.2001
(51) Int. Cl.: A61K 31/4025, A61K 38/28, A61P 3/10

(54) **Combinations comprising dipeptidylpeptidase-IV inhibitors and antidiabetic agents**
Zusammensetzungen bestehend aus Dipeptidylpeptidase-IV Inhibitoren und Antidiabetica
Combinaisons à base d'inhibiteurs de DPP-IV et d'antidiabetiques

(30) Priority: 21.01.2000 US 489234; 19.07.2000 US 619262
(43) Date of publication of application: 10.01.2007
(62) Divisional of application: 01909661.9
(73) Proprietor: Novartis AG, 4002 Basel (CH); Novartis Pharma GmbH, 1230 Wien (AT)
(72) Inventor: Balkan, Börk, Madison, NJ 06443 (US); Hughes, Thomas Edward, Concord, MA 01742 (US); Holmes, David Grenville, 4102, Binningen (CH); Villhauer, Edwin Bernard, Morristown, NJ 07960 (US)
(74) Representative: Gabarda Ortega, Ana Esther

(56) References cited:
- WO-A-98/19998
- WO-A-99/38501
- WO-A-99/61431
- PAULY R P ET AL: "IMPROVED GLUCOSE TOLERANCE IN RATS TREATED WITH THE DIPEPTIDYL PEPTIDASE IV (CD26) INHIBITOR ILE-THIAZOLIDIDE" METABOLISM, CLINICAL AND EXPERIMENTAL, W.B. SAUNDERS CO., PHILADELPHIA, PA, US, vol. 48, no. 3, 1999, pages 385-389, XP000874051 ISSN: 0026-0495
- DEACON C F ET AL: "DIPEPTIDYL PEPTIDASE IV INHIBITION POTENTIATES THE INSULINOTROPIC EFFECT OF GLUCAGON-LIKE PEPTIDE 1 IN THE ANESTHETIZED PIG" DIABETES, NEW YORK, NY, US, vol. 47, no. 5, May 1998 (1998-05), pages 764-769, XP000853618 ISSN: 0012-1797
- DUNNING B E: "NATEGLINIDE: A GLUCOSE-SENSITIVE INSULINOTROPIC AGENT THAT IS CHEMICALLY AND PHARMACOLOGICALLY DISTINCT FROM THE SULFONYLUREAS" CURRENT OPINION IN ENDOCRINOLOGYAND DIABETES, PHILADELPHIA, PA, US, vol. 6, no. SUPPL 1, August 1999 (1999-08), pages S29-S31, XP000997507 ISSN: 1068-3097
- BALKAN B ET AL: "INHIBITION OF DIPEPTIDYL PEPTIDASE IV WITH NVP-DPP728 INCREASES PLASMA GLP-1 (7-36 AMIDE) CONCENTRATIONS AND IMPROVES ORAL GLUCOSE TOLERANCE IN OBESE ZUCKER RATS" DIABETOLOGIA, BERLIN, DE, vol. 42, no. 11, November 1999 (1999-11), pages 1324-1331, XP000921066 ISSN: 0012-186X
- EDELMAN S V: "Type II diabetes mellitus." ADVANCES IN INTERNAL MEDICINE. 1998, vol. 43, 1998, pages 449-500, XP009074961 ISSN: 0065-2822
- HOLST J J ET AL: "INHIBITION OF THE ACTIVITY OF DIPEPTIDYL-PEPTIDASE IV AS A TREATMENT FOR TYPE 2 DIABETES" DIABETES, NEW YORK, NY, US, vol. 47, November 1998 (1998-11), pages 1663-1670, XP000853619 ISSN: 0012-1797
- NOVARTIS A G: "NOVEL N-SUBSTITUTED-2-CYANOPYRROLIDINES AS POTENT INHIBITORS OF DIPEPTIDYL PEPTIDASE IV IN THE TREATMENT OF NON-INSULIN-DEPENDENT DIABETES MELLITUS" EXPERT OPINION ON THERAPEUTIC PATENTS, ASHLEY PUBLICATIONS, GB, vol. 10, no. 12, December 2000 (2000-12), pages 1937-1942, XP001019155 ISSN: 1354-3776

## Description

The invention relates to a combination, such as a combined preparation or pharmaceutical composition, respectively, which comprises a dipeptidylpeptidase - IV (DPP-IV) inhibitor and at least one further antidiabetic compound, consisting of

insulin, for simultaneous, separate or sequential use, especially in the prevention, delay of progression or treatment of conditions mediated by dipeptidylpeptidase - IV (DPP-IV), in particular diabetes, more particular type 2 diabetes mellitus, conditions of impaired glucose tolerance (IGT), conditions of impaired fasting plasma glucose, metabolic acidosis, ketosis, and obesity; the use of such combination for the preparation of a pharmaceutical preparation for the prevention, delay of progression or treatment of such conditions.

DPP-IV is responsible for inactivating GLP-1. More particularly, DPP-IV generates a GLP-1 receptor antagonist and thereby shortens the physiological response to GLP-1. GLP-1 is a major stimulator of pancreatic insulin secretion and has direct beneficial effects on glucose disposal.

Non-insulin dependent diabetes mellitus (type 2 diabetes mellitus) is characterized by both increased peripheral insulin resistance and abnormal insulin secretion. At least three abnormalities of insulin secretion are recognized: in the first phase, insulin secretion is lost and in the second phase insulin is both delayed and inadequate in the face of elevated circulating glucose levels. Several metabolic, hormonal, and pharmacological entities are known to stimulate insulin secretion including glucose, amino-acids and gastrointestinal peptides. The Diabetes Control and Complications Trial (DCCT) has established that lowering of blood glucose is associated with decreases in the onset and progression of diabetic microvascular complications (Diabetes Control and Complications Trial Research Group; N. Engl. J. Med. 1993, 329, 977-986). IGT is an impairment of glucose homeostasis closely related to type 2 diabetes mellitus. Both conditions convey a great risk of macrovascular disease. Therefore, one therapeutic focus is on optimizing and potentially normalizing glycemic control in subjects with type 2 diabetes mellitus, conditions of impaired fasting plasma glucose, or IGT. Presently available agents need to be improved in order to better meet this therapeutic challenge.

The present invention relates to a combination which comprises a DPP-IV inhibitor according to claim 1 in free or pharmaceutically acceptable salt form, and insulin or the pharmaceutically acceptable salt of such a compound and optionally at least one pharmaceutically acceptable carrier; for simultaneous, separate or sequential use.

The DPP-IV inhibitor according to the invention is non-peptidic.

DPP-IV inhibitors are generically and specifically disclosed in WO 98/19998, DE 196 16 486 A1, WO 00/34241 and WO 95/15309.

DPP728 and LAF237 are specifically disclosed in Example 3 of WO 98/19998 and Example 1 of WO 00/34241, respectively.

Comprised are likewise the corresponding stereoisomers as well as the corresponding polymorphs, e.g. crystal modifications, which are disclosed in the cited patent documents.

The DPP-IV inhibitor is (S)-1-[(3-hydroxy-1-adamantyl)amino]acetyl-2-cyano-pyrrolidine.

The term "prevention" means prophylactic administration of the combination to healthy patients to prevent the outbreak of the conditions mentioned herein. Moreover, the term "prevention" means prophylactic administration of such combination to patients being in a pre-stage of the conditions, especially diabetes, to be treated.

The term "delay of progression" used herein means administration of the combination, such as a combined preparation or pharmaceutical composition, to patients being in a pre-stage of the condition, especially diabetes, to be treated in which patients a pre-form of the corresponding condition is diagnosed.

The structure of the active agents identified by code nos., generic or trade names may be taken from the actual edition of the standard compendium "The Merck Index" or from databases, e.g. Patents International (e.g. IMS World Publications). The corresponding content thereof is hereby incorporated by reference. Any person skilled in the art is fully enabled to identify the active agents and, based on these references, likewise enabled to manufacture and test the pharmaceutical indications and properties in standard test models, both *in vitro* and *in vivo.*

The compounds to be combined can be present as pharmaceutically acceptable salts. If these compounds have, for example, at least one basic center, they can form acid addition salts. Corresponding acid addition salts can also be formed having, if desired, an additionally present basic center. The compounds having an acid group (for example COOH) can also form salts with bases. For example, the compounds to be combined can be present as a sodium salt, as a maleate or as a dihydrochloride. The active ingredient or a pharmaceutically acceptable salt thereof may also be used in form of a hydrate or include other solvents used for crystallization.

The antidiabetic compound, insulin, or a pharmaceutically acceptable salt of such a compound, will be referred to hereinafter as COMBINATION PARTNER OF THE INVENTION.

A combined preparation which comprises a DPP-IV inhibitor in free or pharmaceutically acceptable salt form and at least one further COMBINATION PARTNER OF THE INVENTION and optionally at least one, i.e., one or more, e.g. two, pharmaceutically acceptable carrier for simultaneous, separate or sequential use is especially a "kit of parts" in the sense that the components, a DPP-IV inhibitor in free or pharmaceutically acceptable salt form and at least one further COMBINATION PARTNER OF THE INVENTION, can be dosed independently or by use of different fixed combinations with distinguished amounts of the components, i.e. at different time points or simultaneously. The parts of the kit of parts can then, e.g., be administered simultaneously or chronologically staggered, that is at different time points and with equal or different time intervals for any part of the kit of parts. Preferably, the time intervals are chosen such that the effect on the treated disease or condition in the combined use of the parts is larger than the effect which would be obtained by use of only any one of the components. Preferably, there is at least one beneficial effect, e.g. a mutual enhancing of the effect of a DPP-IV inhibitor in free or pharmaceutically acceptable salt form, and at least one further COMBINATION PARTNER OF THE INVENTION, additional advantageous effects, less side effects, a combined therapeutical effect in a non-effective dosage of one or each of the components, and especially a synergism, e.g. a more than additive effect, between a DPP-IV inhibitor in free or pharmaceutically acceptable salt form, and at least one further COMBINATION PARTNER OF THE INVENTION.

The nature of conditions mediated by DPP-IV, especially diabetes, conditions of impaired fasting plasma glucose, and IGT, is multifactorial. Under certain circumstances, drugs with different mechanisms of action may be combined. However, just considering any combination of drugs having different mode of action but acting in the similar field does not necessarily lead to combinations with advantageous effects.

All the more surprising is the experimental finding that the combined administration of a DPP-IV inhibitor and at least one further COMBINATION PARTNER OF THE INVENTION results not only in a beneficial, especially a synergistic, therapeutic effect but also in additional benefits resulting from combined treatment such as a surprising prolongation of efficacy, a broader variety of therapeutic treatment and surprising beneficial effects on diseases and conditions associated with diabetes, e.g. less gain of weight.

Further benefits are that lower doses of the individual drugs to be combined according to the present invention can be used to reduce the dosage, for example, that the dosages need not only often be smaller but are also applied less frequently, or can be used in order to diminish the incidence of side effects. This is in accordance with the desires and requirements of the patients to be treated.

It can be shown by established test models and especially those test models described herein that the combination of the DPP-IV inhibitor, (S)-1-[(3-hydroxy-1-adamantyl)amino]acetyl-2-cyano-pyrrolidine (LAF237), and at least one further COMBINATION PARTNER OF THE INVENTION results in a more effective prevention or preferably treatment of conditions mediated by DPP-IV, in particular diabetes, especially type 2 diabetes mellitus, conditions of impaired fasting plasma glucose, and conditions of IGT.

The person skilled in the pertinent art is fully enabled to select a relevant animal test model to prove the hereinbefore and hereinafter indicated therapeutic indications and beneficial effects. The pharmacological activity may, for example, be demonstrated following essentially an *in-vivo* test procedure in mice or in a clinical study as described hereinafter.

### In-vivo test in mice for blood glucose control

ICR-CDI mice (male, five weeks old, body weight: about 20 g) are abstained from food for 18 hours, and then used as test subjects. The combination according to the present invention and the active ingredients alone are suspended in 0.5% CMC-0.14M sodium chloride buffer solution (pH 7.4). The solution thus obtained is administered orally in fixed volume amounts to the test subjects. After predetermined time, the percentage decrease of the blood glucose against the control group is determined.

### Clinical double-blind, randomized, parallel-group study in subjects with type 2 diabetes mellitus inadequately controlled on diet alone

This study proves in particular the synergism of a combined preparation or pharmaceutical composition, respectively. The beneficial effects on conditions mediated by DPP-IV, in particular type 2 diabetes mellitus can be determined directly through the results of this study or by changes in the study design which are known as such to a person skilled in the art.

The study is, in particular, suitable to compare the effects of monotherapy with a COMBINATION PARTNER OF THE INVENTION with those of a combination of DPP-IV inhibitor plus one of these compounds on glycemic control.

Subjects with a diagnosis of type 2 diabetes mellitus who have not achieved near normoglycemia (HbA_{1c} <6.8%) on diet only are chosen for this trial. The effects on glycemic control achieved with DPP-IV monotherapy, monotherapy with one COMBINATION PARTNER OF THE INVENTION, and the combination therapy of DPP-IV plus one COMBINATION PARTNER OF THE INVENTION are determined in this study after 24 weeks with the control achieved on placebo, all subjects continuing with the same diet as in the period before treatment. Measures of glycemic control are validated surrogate endpoints for the treatment of diabetes. HbA_{1c} is the single most reliable measurement for assessing glycemic control (D. Goldstein et al, Tests of Glycemia in Diabetes; Diabetes Care 1995, 18(6), 896-909) and is the primary response variable in this study. Since glycosylation of hemoglobin is determined by the glucose concentration at the time each red blood cell is made, HbA_{1c} provides an estimate of mean blood glucose for the previous three months.

Before starting with the double-blind treatment for 24 weeks, the subjects are administered for four weeks the placebos matching with the DPP-IV inhibitor, e.g. DPP728 and LAF237, before breakfast, lunch and dinner, and the placebos matching with one or more of the COMBINATION PARTNERS OF THE INVENTION (period I). For example, if the α-glucosidase inhibitors acarbose is chosen for the study, the placebo matching with acarbose is preferably administered together with the first bite of the meals taken for breakfast, lunch and dinner in period I. If the antidiabetic phenylacetic acid derivative repaglinide is chosen for the study, the placebos matching with repaglinide are preferably administered later on with breakfast, lunch and dinner in period I. If the antidiabetic thiazolidinedione troglitazone is chosen for the study, the placebos matching with troglitazone are preferably administered in period I with breakfast only. If the antidiabetic D-phenylalanine derivative nateglinide is chosen for the study, matching placebos are preferably administered before breakfast, lunch and dinner period I. If metformin is chosen for the study, matching placebos are preferably administered before breakfast and dinner.

The subjects are then separated into four treatment groups for the 24-week double-blind study (period II) as depicted in Tables 1 to 5 for the case that DPP728 is chosen as the DPP-IV inhibitor and one of the drugs comprising the antidiabetic thiazolidinedione troglitazone, the antidiabetic phenylacetic acid derivative repaglinide, the α-glucosidase inhibitor acarbose, the antidiabetic D-phenylalanine derivative nateglinide or the biguanide metformin is chosen as the combination partner.

### Examples for Combinations to be administered

**Table 1: DPP728 plus troglitazone**

| |
|---|
| DPP728 50 mg* + troglitazone placebo** |
| troglitazone 600 mg** + DPP728 placebo* |
| DPP728 50 mg* + troglitazone 600 mg** |
| DPP728 placebo* + troglitazone placebo** |

| |
|---|
| * administered before breakfast, lunch, and dinner; ** administered once daily with breakfast |

**Table 2: DPP728 plus repaglinide**

| |
|---|
| DPP728 50 mg* + repaglinide placebo* |
| repaglinide 1 mg* + DPP728 placebo* |
| DPP728 50 mg* + repaglinide 1 mg* |
| DPP728 placebo* + repaglinide placebo* |

| |
|---|
| * administered before breakfast, lunch, and dinner |

**Table 3: DPP728 plus acarbose**

| |
|---|
| DPP728 50 mg* + acarbose placebo** |
| acarbose 50 mg** + DPP728 placebo* |
| DPP728 50 mg* + acarbose 50 mg** |
| DPP728 placebo* + acarbose placebo** |

| |
|---|
| * administered before breakfast, lunch, and dinner ** administered together with the first bite of breakfast, lunch and dinner |

**Table 4: DPP728 plus nateglinide**

| |
|---|
| nateglinide (I) 120 mg* + DPP728 placebo* |
| DPP728 50 mg* + nateglinide (I) placebo* |
| nateglinide (I) 120 mg* + DPP728 50 mg* |
| nateglinide (I) placebo* + DPP728 placebo* |

| |
|---|
| * administered before breakfast, lunch, and dinner |

**Table 5: DPP728 plus metformin**

| |
|---|
| metformin 500 mg** + DPP728 placebo* |
| DPP728 50 mg* + metformin placebo** |
| metformin 500 mg** + DPP728 50 mg* |
| metformin placebo** + DPP728 placebo* |

| |
|---|
| * administered before breakfast, lunch, and dinner ** administered before breakfast and dinner |

DPP728 tablets contain either 50 mg of the compound or matching placebo. Nateglinide tablets contain either 120 mg or matching placebo. Troglitazone 200 mg tablets, repaglinide 1 mg tablets, acarbose 50 mg tablets and metformin 500 mg tablets can be purchased commercially and overencapsulated to match the corresponding placebo capsules.

The subjects are then separated into four treatment groups for the 24-week double-blind study (period II) as depicted in Table 1. Approximately 170 subjects are randomized per treatment group. The total study duration including the run-in period for each subject is 28 weeks. Statistical analysis can be carried out by methods known in the art.

The subject is advised not to take the morning dose of study medication or eat breakfast on the day of a scheduled study visit. The morning dose is administered by site personnel after the collection of all fasting laboratory samples and completion of all study procedures. Visits are scheduled to be performed at 2 week intervals during period I, and 4 to 8 week intervals during period II. Subjects have fasted for at least 7 hours at the time of each visit. All blood samples for laboratory evaluations are drawn between 7:00 AM and 10:00 AM. All tests are conducted in accordance with Good Laboratory Practice principles following procedures known in the art.

HbA_{1c} is measured by High Performance Liquid Chromatography (HPLC) using the ionexchange method on a Bio-Rad Diamat analyzer. A back-up affinity method are used if hemoglobin variants or hemoglobin degradation peaks are observed.

Further parameters to be determined are fasting plasma glucose (FPG), fasting lipids (total, HDL (high density lipoprotein)- and LDL (low density lipoprotein)-cholesterol, and triglycerides) and body weight. FPG will be measured using the hexokinase method and LDL-cholesterol will be calculated using the Friedewald formula if triglycerides are < 400 mg/dL (4.5 mmol/l).

Various parameters of the study described above can be modified, e.g. in order to optimize the dosage for special diseases or indications mentioned herein, to cope with tolerability problems during the study or to obtain similar or identical results with less efforts. For example, a different subject population can be involved in such a clinical trial, e.g. subjects with a diagnosis of type 2 diabetes mellitus who have achieved near normoglycemia (HbA_{1c} <6.8%) on diet alone, subjects with diseases other than diabetes mellitus, e.g. other metabolic disorders, or subjects selected by other criteria, such as age or sex; the subject number can be decreased, e.g. to a number of between 70 and 150, especially 100 or 120, subjects per treatment group; treatment groups (listed exemplary in Table 1) can be deleted, i.e. for example to carry out a study with a comparison of the combination of a DPP-IV inhibitor and at least one further COMBINATION PARTNER OF THE INVENTION versus a DPP-IV inhibitor alone; the term of the placebo run-in period (period I) can be changed, i.e. it can be extended, shortened or deleted; the visit schedule can be extended, e.g. to every 10, 12 or 14 weeks; the visit instructions can be changed, e.g. the instruction that blood samples for laboratory evaluations have to be drawn between 7:00 AM and 10:00 AM; HbA_{1c} can be determined by other means; or one or more of the parameters to be determined during the study mentioned above, e.g. FPG or fasting lipids, can be deleted or the determination of additional parameters (see below) can be added.

Additional parameters can be determined in the course of the study, e.g. by additional tests. Such additional tests can comprise the analysis of body liquids in order to determine amounts or numbers for parameters such as those listed below and can serve e.g. the purpose of determining the tolerability of the administered active ingredients: determination of hematocrit and hemogloblin, platelet count, erythrocyte count, total and differential leukocyte count (basophils, eosinophils, lymphocytes, monocytes, segmented neutrophils and total neutrophils); determination of albumin, alkaline phosphatase, alanine amino transferase (serum glutamic pyruvic transaminase), aspartate amino transferase (serum glutamic oxaloacetic transaminase), blood urea nitrogen or urea, bicarbonate, calcium, chloride, total creatine phosphokinase (CPK), creatine phosphokinase muscle-brain fraction isoenzyme (if CPK is elevated), direct bilirubin, creatinine, γ-glutamyl transferase, lactate dehydrogenase, potassium, sodium, total bilirubin, total protein and uric acid in the blood; determination of bilirubin, glucose, ketones, pH, protein, and specific gravity in the subjects urine; determination of body weight, blood pressure (systolic and diastolic, after 3 minutes sitting) and radial pulse (after 3 minutes sitting).

The results of the studies show that the combination according to the present invention can be used for the prevention and preferably the treatment of conditions mediated by DPP-IV, in particular type 2 diabetes mellitus. The combination of the present invention can also be used for the prevention and preferably the treatment of other condition mediated by DPP-IV.

Furthermore, in a number of combinations as disclosed herein the side-effects observed with one of the components surprisingly do not accumulate on application of the combination. Preferably, the jointly therapeutically effective amounts of a DPP-IV inhibitor in free or pharmaceutically acceptable salt form and at least one further pharmaceutically active compound are administered simultaneously or sequentially in any order, separately or in a fixed combination.

The condition mediated by DPP-IV is preferably selected from the group consisting of diabetes, impaired fasting plasma glucose, impaired glucose tolerance, metabolic acidosis, ketosis, arthritis, obesity and osteoporosis.

Very preferably, the condition mediated by DPP-IV is type 2 diabetes mellitus.

It is one objective of this invention to provide a pharmaceutical composition comprising a quantity, which is jointly therapeutically effective against conditions mediated by DPP-IV, in particular diabetes, more especially type 2 diabetes mellitus, conditions of impaired fasting plasma glucose, and conditions of IGT, of a DPP-IV inhibitor (i) or a pharmaceutically acceptable salt thereof and (ii) at least one further COMBINATION PARTNER OF THE INVENTION and at least one pharmaceutically acceptable carrier.

The pharmaceutical compositions according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carries, especially suitable for enteral or parenteral application.

The novel pharmaceutical preparations contain, for example, from about 10 % to about 100 %, e.g., 80% or 90 %, preferably from about 20 % to about 60 %, of the active ingredient. Pharmaceutical preparations according to the invention for enteral or parenteral administration are, for example, those in unit dose forms, such as sugar-coated tablets, tablets, capsules or suppositories, and furthermore ampoules. These are prepared in a manner known per se, for example by means of conventional mixing, granulating, sugar-coating, dissolving or lyophilizing processes. Thus, pharmaceutical preparations for oral use can be obtained by combining the active ingredient with solid carriers, if desired granulating a mixture obtained, and processing the mixture or granules, if desired or necessary, after addition of suitable excipients to give tablets or sugar-coated tablet cores.

In this composition, components (i) and (ii) can be administered together, one after the other or separately in one combined unit dose form or in two separate unit dose forms. In one preferred embodiment of the invention, the unit dose form is a fixed combination. In a fixed combination the components (i) and (ii) are administered in the form of a single galenic formulation, e.g. a single tablet or a single infusion.

A further aspect of the present invention is the use of a pharmaceutical composition comprising a DPP-IV inhibitor and at least one further COMBINATION PARTNER OF THE INVENTION, in each case in free form or in form of a pharmaceutically acceptable salt thereof for the preparation of a pharmaceutical preparation for the prevention or treatment of conditions mediated by DPP-IV, in particular diabetes, more especially type 2 diabetes mellitus, conditions of impaired fasting plasma glucose, and conditions of IGT.

A therapeutically effective amount of each of the components of the combination of the present invention may be administered simultaneously or sequentially and in any order, and the components may be administered separately or as a fixed combination. For example, the method of treatment of the invention may comprise (i) administration of a DPP-IV inhibitor in free or pharmaceutically acceptable salt form and (ii) adminstration of at least one further COMBINATION PARTNER OF THE INVENTION simultaneously or sequentially in any order, in jointly therapeutically effective amounts, preferably in synergistically effective amounts, e.g. in daily dosages corresponding to the ratios described herein.

The corresponding active ingredient or a pharmaceutically acceptable salt thereof may also be used in form of a hydrate or include other solvents used for crystallization.

Furthermore, the term administering also encompasses the use of prodrugs of any of the anti-diabetic drugs that convert in vivo to the selective anti-diabetic drug. The instant invention is therefore to be understood as embracing all such regimes of simultaneous or alternating treatment and the term "administering" is to be interpreted accordingly.

If the combination comprises nateglinide, a composition, in particular a pharmaceutical composition, comprising solely nateglinide can be produced by a process that comprises granulating in the presence of water to form granules, drying the granules, and optionally screening the granules, for example, through a wire mesh screen. All of the ingredients of the composition may be added prior to or during the granulation. Alternatively, all or a portion of one or more of the ingredients may be added after the granulation step is complete. For example, all or a portion of anti-adherent (e.g., silica), all or a portion of lubricant (e.g., magnesium stearate) and/or all or a portion of disintegrant (e.g., croscarmellose or any salt thereof) may be added after the granulation. In one aspect of the invention, all ingredients except the magnesium stearate and the colloidal silica are loaded into the granulator, then they are added later. The process of producing this composition, in particular pharmaceutical composition, may be performed without the need for a pulverization step. As used herein, the terms "pulverization" and "pulverize" refer to any process that involves the grinding or smashing cutting of particles to reduce the particles' size. The composition, in particular pharmaceutical composition, is capable of being produced without pulverizing the granules between the granulation step and the drying and/or compression step used to form the granules into a tablet. In one preferred embodiment of the invention, nateglinide is used in the B-type or H-type crystal modification.

A further aspect of the present invention is a method of treating a condition mediated by DPP-IV, in particular type 2 diabetes mellitus, comprising administering to a warm-blooded animal in need thereof jointly therapeutically effective amounts of a DPP - IV inhibitor in free or pharmaceutically acceptable salt form, and at least one further COMBINATION PARTNER OF THE INVENTION. Preferably, in this method of treating the active ingredients are administered simultaneously or sequentially in any order, separately or in a fixed combination. In one preferred embodiment of such method the jointly therapeutically effective amounts of a dipeptidylpeptidase - IV inhibitor in free or pharmaceutically acceptable salt form and at least one further COMBINATION PARTNER OF THE INVENTION are provided as a combined preparation.

Furthermore, the present invention provides a method of treating conditions of impaired glucose tolerance and impaired fasting plasma glucose comprising administering to a warm-blooded animal in need thereof jointly therapeutically effective amounts of a DPP - IV inhibitor in free or pharmaceutically acceptable salt form, and at least one further COMBINATION PARTNER OF THE INVENTION.

The dosage range of the combination of a DPP-IV inhibitor and at least one further COMBINATION PARTNER OF THE INVENTION to be employed depends upon factors known to the person skilled in the art including species of the warm-blooded animal, body weight and age, the nature and severity of the condition to be treated, the mode of administration and the particular substance to be employed. Unless stated otherwise herein, the DPP-IV inhibitor and at least one further COMBINATION PARTNER OF THE INVENTION are preferably divided and administered from one to four times per day.

The weight ratio of the daily doses of LAF237 or a pharmaceutically acceptable salt thereof to at least one further COMBINATION PARTNER OF THE INVENTION may vary within wide limits depending in particular on the needs of the warm-blooded animal treated.

The following Reference Examples shall illustrate the disclosure described above.

### Reference Example 1: Tablets of Nateglinide

108,000 tablets, each which contain 120 mg of nateglinide are prepaired as follows:

| | | |
|---|---|---|
| Composition: | nateglinide | 12.960 kg |
| | lactose, NF | 30.564 kg |
| | microcrystalline cellulose, NF | 15.336 kg |
| | povidone, USP | 2.592 kg |
| | croscarmellose sodium, NF | 3.974 kg |
| | colloidal silicon dioxide, NF | 1.382 kg |
| | magnesium stearate, NF | 1.231 kg |
| | coating: opadry yellow | 1.944 kg |
| | purified water, USP* | Q.S. |

| | | |
|---|---|---|
| *: removed during process | | |

Preparation process: The microcrystalline cellulose, povidone, part of the croscarmellose sodium, nateglinide and lactose are mixed in a high shear mixer and afterwards granulated using purified water. The wet granules are dried in a fluid bed dryer and passed through a screen. The colloidal silicon dioxide and the rest of the croscarmellose sodium are mixed, passed through a screen and blended with the dried granules in a V-blender. The magnesium stearate is passed through a screen, blended with the blend from the V-blender and afterwards the total mixture is compressed to tablets. The opadry yellow is suspended in purified water and the tablets are coated with the coating suspension.

### Reference Example 2: Galenic Formulation of Nateglinide No. 1

| intra-granular: | |
|---|---|
| nateglinide | 120 mg |
| lactose monohydrate | 283 mg |
| microcrystalline cellulose | 142 mg |
| povidone | 24 mg |
| croscarmellose sodium | 24 mg |

| extra-granular: | |
|---|---|
| magnesium stearate | 7 mg |
| opadry white | 20 mg |

### Reference Example 3: Galenic Formulation of Nateglinide No.2

| intra-granular: | |
|---|---|
| nateglinide | 120 mg |
| lactose monohydrate | 283 mg |
| microcrystalline cellulose | 142 mg |
| povidone | 24 mg |
| croscarmellose sodium | 24 mg |

| extra-granular: | |
|---|---|
| croscarmellose sodium | 12.8 mg |
| magnesium stearate | 11.4 mg |
| opadry yellow | 18.0 mg |
| colloidal silicon dioxide | 12.8 mg |

### Reference Example 4: Tablets of Nateglinide

108,000 tablets, each which contain 120 mg of nateglinide are prepared as follows:

| | | |
|---|---|---|
| Composition: | nateglinide | 12.960 kg |
| | lactose, NF | 30.564 kg |
| | microcrystalline cellulose, NF | 15.336 kg |
| | povidone, USP | 2.592 kg |
| | croscarmellose sodium, NF | 3.974 kg |
| | colloidal silicon dioxide, NF | 1.382 kg |
| | magnesium stearate, NF | 1.231 kg |
| | coating: opadry yellow | 1.944 kg |
| | purified water, USP* | Q.S. |

| | | |
|---|---|---|
| *: removed during process | | |

Preparation process: The microcrystalline cellulose, povidone, a portion of the croscarmellose sodium, nateglinide and lactose are granulated in a collette gral granulator with the addition of purified water. The wet granules are dried in a fluid bed dryer and passed through a screen. The colloidal silicon dioxide and the rest of the croscarmellose sodium are mixed, passed through a screen and blended with the dried granules in a V-blender. The magnesium stearate is passed through a screen, blended with the blend from the V-blender and afterwards the total mixture is compressed to tablets. The opadry yellow is suspended in purified water and the tablets are coated with the coating suspension. Variants of this process include adding the colloidal silica and the remaining croscarmellose sodium to the second granulator load after drying, then screening together; and combining as many as 3 granulator/drier loads per batch.

### Reference Example 5: Pharmaceutical composition of Nateglinide (120 mg)

| | |
|---|---|
| nateglinide | 120 mg |
| lactose monohydrate | 283 mg |
| microcrystalline cellulose | 142 mg |
| Povidone | 24 mg |
| croscarmellose sodium | 36.8 mg |
| magnesium stearate | 11.4 mg |
| opadry yellow | 18.0 mg |
| colloidal silicon dioxide | 12.8 mg |

## Claims

1. A combination comprising a dipeptidylpeptidase - IV inhibitor (DPP-IV inhibitor) which is (S)-1-[(3-hydroxy-1-adamantyl)amino]acetyl-2-cyano-pyrrolidine,
in free form or in acid addition salt form, and
insulin or the pharmaceutically acceptable salt of such a compound.

2. Combination according to claim 1 which is a combined preparation for simultaneous, separate or sequential use in the prevention, delay of progression or treatment of conditions mediated by DPP-IV.

3. Combination according to claim 2 wherein the conditions mediated by DPP-IV are selected from diabetes, type 2 diabetes mellitus, conditions of impaired glucose tolerance (IGT), conditions of impaired fasting plasma glucose, metabolic acidosis, ketosis and obesity.

4. Combination according to any one of claims 1 to 3 for use as a medicament.

5. Use of a combination according to any one of claims 1 to 3 for the preparation of a medicament for the prevention, delay of progression or treatment of a condition selected form diabetes, type 2 diabetes mellitus, conditions of impaired glucose tolerance (IGT), conditions of impaired fasting plasma glucose, metabolic acidosis, ketosis and obesity.

6. Combination according to claim 2 which is contained in a commercial package together with instructions for simultaneous, separate or sequential use thereof in the prevention, delay of progression or treatment of a condition mediated by DPP-IV.

7. Use of a dipeptidylpeptidase - IV inhibitor (DPP-IV inhibitor) which is (S)-1-[(3-hydroxy-1-adamantyl)amino]acetyl-2-cyano-pyrrolidine, in free form or in acid addition salt form, in combination with insulin or a pharmaceutically acceptable salt thereof, for the preparation of a medicament for the prevention, delay of progression or treatment of a condition selected form diabetes, type 2 diabetes mellitus, conditions of impaired glucose tolerance (IGT), conditions of impaired fasting plasma glucose, metabolic acidosis, ketosis and obesity.

8. Use according to claim 7, for the preparation of a medicament for the prevention, delay of progression or treatment of diabetes.

9. Use according to claim 7, for the preparation of a medicament for the prevention, delay of progression or treatment of type 2 diabetes mellitus.

10. A combination **according to claim** 1 for use in the prevention, delay of progression or treatment of a condition selected form diabetes, type 2 diabetes mellitus, conditions of impaired glucose tolerance (IGT), conditions of impaired fasting plasma glucose, metabolic acidosis, ketosis and obesity.

11. A combination **according to claim 10** for use in the prevention, delay of progression or treatment of diabetes.

12. A combination **according to claim 11** for use in the prevention, delay of progression or treatment of type 2 diabetes mellitus.

## Patentansprüche

1. Kombination, umfassend einen Dipeptidylpeptidase-IV-Inhibitor (DPP-IV-Inhibitor), nämlich (S)-1-[(3-Hydroxy-1-adamantyl)amino]acetyl-2-cyano-pyrrolidin in freier Form oder in Form eines Säureadditionssalzes und Insulin oder das pharmazeutisch akzeptable Salz einer solchen Verbindung.

2. Kombination gemäß Anspruch 1, die eine kombinierte Zubereitung für die gleichzeitige, getrennte oder sequentielle Verwendung bei der Prävention, Verzögerung der Progression oder Behandlung von durch DPP-IV vermittelten Zuständen ist.

3. Kombination gemäß Anspruch 2, wobei die durch DPP-IV vermittelten Zustände aus Diabetes, Diabetes mellitus Typ 2, Zuständen einer beeinträchtigten Glukosetoleranz (IGT), Zuständen einer beeinträchtigten Fastenplasmaglukose, metabolischer Acidose, Ketose und Fettleibigkeit ausgewählt sind.

4. Kombination gemäß einem der Ansprüche 1 bis 3 zur Verwendung als ein Medikament.

5. Verwendung einer Kombination gemäß einem der Ansprüche 1 bis 3 zur Herstellung eines Medikaments zur Prävention, Verzögerung der Progression oder Behandlung eines Zustands der aus Diabetes, Diabetes mellitus Typ 2, Zuständen einer beeinträchtigten Glukosetoleranz (IGT), Zuständen einer beeinträchtigten Fastenplasmaglukose, metabolischer Acidose, Ketose und Fettleibigkeit ausgewählt ist.

6. Kombination gemäß Anspruch 2, die in einer kommerziellen Packung zusammen mit Anleitungen zur gleichzeitigen, getrennten oder sequenziellen Verwendung hiervon bei der Prävention, Verzögerung der Progression oder Behandlung eines Zustands, der durch DPP-IV vermittelt wird, enthalten ist.

7. Verwendung eines Dipeptidylpeptidase-IV-Inhibitors (DPP-IV-Inhibitors), nämlich (S)-1-[(3-Hydroxy-1-adamantyl)amino]acetyl-2-cyano-pyrrolidin in freier Form oder in Form eines Säureadditionssalzes, in Kombination mit Insulin oder einem pharmazeutisch akzeptablen Salz hiervon zur Herstellung eines Medikaments zur Prävention, Verzögerung der Progression oder Behandlung eines Zustands, der aus Diabetes, Diabetes mellitus Typ 2, Zuständen einer beeinträchtigten Glukosetoleranz (IGT), Zuständen einer beeinträchtigten Fastenplasmaglukose, metabolischer Acidose, Ketose und Fettleibigkeit ausgewählt ist.

8. Verwendung gemäß Anspruch 7 zur Herstellung eines Medikaments zur Prävention, Verzögerung der Progression oder Behandlung von Diabetes.

9. Verwendung gemäß Anspruch 7 zur Herstellung eines Medikaments zur Prävention, Verzögerung der Progression oder Behandlung von Diabetes mellitus Typ 2.

10. Kombination gemäß Anspruch 1 zur Verwendung bei der Prävention, Verzögerung der Progression oder Behandlung eines Zustands, der aus Diabetes, Diabetes mellitus Typ 2, Zuständen einer beeinträchtigten Glukosetoleranz (IGT), Zuständen einer beeinträchtigten Fastenplasmaglukose, metabolischer Acidose, Ketose und Fettleibigkeit ausgewählt ist.

11. Kombination gemäß Anspruch 10 zur Verwendung bei der Prävention, Verzögerung der Progression oder Behandlung von Diabetes.

12. Kombination gemäß Anspruch 11 zur Verwendung bei der Prävention, Verzögerung der Progression oder Behandlung von Diabetes mellitus Typ 2.

## Revendications

1. Combinaison comprenant un inhibiteur de dipeptidylpeptidase-IV (inhibiteur de DPP-IV) qui est la (S)-1-[(3-hydroxy-1-adamantyl)amino]acétyl-2-cyano-pyrrolidine, sous forme libre ou sous forme de sel d'addition d'acide, et de l'insuline ou le sel pharmaceutiquement acceptable d'un tel composé.

2. Combinaison selon la revendication 1, qui est une préparation combinée destinée à une utilisation simultanée, séparée ou séquentielle pour la prévention, pour retarder la progression ou pour le traitement d'affections dépendantes de DPP-IV.

3. Combinaison selon la revendication 2, où les affections dépendantes de DPP-IV sont choisies parmi le diabète, le diabète sucré de type 2, les affections d'intolérance au glucose (IGT), les affections d'hyperglycémie modérée à jeun, l'acidose métabolique, la cétose et l'obésité.

4. Combinaison selon l'une quelconque des revendications 1 à 3, à utiliser comme médicament.

5. Utilisation d'une combinaison selon l'une quelconque des revendications 1 à 3 pour la préparation d'un médicament destiné à la prévention, à retarder la progression ou au traitement d'une affection choisie parmi le diabète, le diabète sucré de type 2, les affections d'intolérance au glucose (IGT), les affections d'hyperglycémie modérée à jeun, l'acidose métabolique, la cétose et l'obésité.

6. Combinaison selon la revendication 2, qui est contenue dans un conditionnement commercial, conjointement avec les instructions relatives à son utilisation simultanée, séparée ou séquentielle pour la prévention, pour retarder la progression ou pour le traitement d'une affection dépendante de DPP-IV.

7. Utilisation d'un inhibiteur de dipeptidylpeptidase-IV (inhibiteur de DPP-IV) qui est la (S)-1-[(3-hydroxy-1-adamantyl)amino]acétyl-2-cyano-pyrrolidine, sous forme libre ou sous forme de sel d'addition d'acide, en combinaison avec de l'insuline ou l'un de ses sels acceptables sur le plan pharmaceutique, pour la préparation d'un médicament destiné à la prévention, à retarder la progression ou au traitement d'une affection choisie parmi le diabète, le diabète sucré de type 2, les affections d'intolérance au glucose (IGT), les affections d'hyperglycémie modérée à jeun, l'acidose métabolique, la cétose et l'obésité.

8. Utilisation selon la revendication 7, pour la préparation d'un médicament destiné à la prévention, à retarder la progression ou au traitement du diabète.

9. Utilisation selon la revendication 7, pour la préparation d'un médicament destiné à la prévention, à retarder la progression ou au traitement du diabète sucré de type 2.

10. Combinaison selon la revendication 1 à utiliser pour la prévention, pour retarder la progression ou pour le traitement d'une affection choisie parmi le diabète, le diabète sucré de type 2, les affections d'intolérance au glucose (IGT), les affections d'hyperglycémie modérée à jeun, l'acidose métabolique, la cétose et l'obésité.

11. Combinaison selon la revendication 10 à utiliser pour la prévention, pour retarder la progression ou pour le traitement du diabète.

12. Combinaison selon la revendication 11 à utiliser pour la prévention, pour retarder la progression ou pour le traitement du diabète sucré de type 2.
